# FASCICULE DE BREVET EUROPEEN

(11) **EP 1 566 168 B1**
(45) Date de publication et mention de la délivrance du brevet: **19.04.2006**
(21) Numéro de dépôt: 04290479.7
(22) Date de dépôt: 23.02.2004
(51) Int. Cl.: A61Q 19/02, A61K 8/49, A61K 8/44

(54) **Compositions dermo-cosmétiques dépigmentantes et leur utilisation**
Hautkosmetische Zubereitungen zur Depigmentierung und deren Verwendung
Dermo-cosmetic compositions for depigmenting and use of it

(43) Date de publication de la demande: 24.08.2005
(73) Titulaire: Laboratoires S.V.R., 91220 Le Plessis Pate (FR)
(72) Inventeur: Besse, Renand Laboratoires S.V.R., 91072 Bondoufle (FR)
(74) Mandataire: Burtin, Jean-François

(56) Documents cités:
- WO-A-03/061768
- US-A- 4 185 119
- US-A- 5 824 327
- ELTZE MANFRID ET AL: "Affinity profile at alpha1- and alpha2-adrenoceptor subtypes and in vitro cardiovascular actions of (+)-boldine" EUROPEAN JOURNAL OF PHARMACOLOGY, vol. 443, no. 1-3, 17 mai 2002 (2002-05-17), pages 151-168, XP001182714 ISSN: 0014-2999

## Description

La présente invention se rapporte à des compositions présentant une activité dépigmentante et à leurs utilisations en dermo-cosmétologie.

Les produits éclaircissants du teint répondent à l'attente générale d'une carnation plus claire et d'un teint plus uniforme, les sujets asiatiques ou africano-américains étant plus particulièrement exposés aux taches pigmentaires diffuses (chloasma, mélasma).

Cependant, les populations caucasiennes sont elles aussi concernées par les taches pigmentaires dites « de sénescence », lorsqu'elles affectent les zones les plus exposées au soleil, mains et visage : ces disgrâces deviennent alors un marqueur, non souhaité, de vieillissement cutané.

Pour ces deux raisons, le marché des correcteurs de pigmentation est à la recherche de produits nouveaux et toujours plus efficaces. Cependant, pour des raisons évidentes de sécurité d'emploi, le contrôle de la mélanogénèse doit rester un phénomène réversible et éviter à tout prix un effet de rebond après arrêt du traitement.

### ART ANTERIEUR

De nombreux produits sont actuellement proposés et, pour la plupart, ce sont des inhibiteurs de l'enzyme clé de la mélanogénèse, la tyrosinase.

Une meilleure connaissance des processus de la mélanogénèse, et en particulier des voies en amont de la régulation de la tyrosinase, ouvre la voie à d'autres actifs plus efficaces et/ou présentant une plus grande régularité d'action.

On sait que l'hyper-pigmentation se manifeste par des taches brunes localisées, provoquées par un excès de mélanine. Les taches mélaniques cutanées, qui s'accentuent ou apparaissent avec l'âge, sont ressenties comme un désagrément esthétique, toujours mal supporté sur le plan psychologique.

Selon leur origine, les hyperpigmentations sont classées en deux grands groupes :

### 1. Hyperpigmentation liée à une hyperactivité ou à une prolifération mélanocytaire :

### a) Les Ephélides

Elles sont communément appelées « taches de rousseurs ». Elles apparaissent durant l'enfance, particulièrement sur les peaux claires et se trouvent préférentielement sur les parties découvertes de l'épiderme. Elles s'intensifient après les expositions solaires.

Histologiquement, le nombre de mélanocytes est normal, mais la quantité de mélanine par mélanocyte est augmentée.

### b) Lentigine ou lentigo solaire

Ce sont des taches arrondies et planes, de très petite taille, de teinte allant du jaune brun au noir, apparaissant durant l'enfance. Il est pratiquement impossible de les différencier des éphélides lorsqu'elles ne sont pas de couleur très foncée.

### c) Lentigo sénile

Ce sont des taches brunes apparaissant sur le dos des mains, les avant-bras et sur le visage après la cinquantaine. Histologiquement, on note une hyperpigmentation mélanique de la couche basale de l'épiderme avec accroissement du nombre de mélanocytes.

### d) Mélasma

C'est une nappe pigmentée s'étendant de façon presque toujours symétrique sur le front et les joues, apparaissant plus volontiers chez les femmes brunes. Il peut apparaître dans trois cas :
- durant la grossesse,
- durant la prise d'oestroprogestatifs (chloasma),
- de manière idiopathique.

### 2. Hyperpigmentation par incontinence pigmentaire:

C'est une hyperpigmentation consécutive à une photosensibilisation. C'est la classique dermatose en « breloque » ou dermite des parfums. Ce sont des taches brunes, plus ou moins foncées, disposées en traînées ou en coulées, aux endroits où sont appliqués les parfums et les cosmétiques, avant une exposition solaire. Elles disparaissent progressivement et spontanément en plusieurs mois.

Il existait donc un besoin pour une composition ayant une activité dépigmentante à faible concentration, présentant une bonne tolérance cutanée.

Pendant longtemps, l'hydroquinone et ses dérivés, comme l'arbutoside ou la méthylarbutine, ont constitué le principe actif essentiel des compositions dépigmentantes mais leur action est fugace, et surtout on constate l'existence d'un effet cytotoxique de l'hydroquinone qui exclut définitivement son utilisation dans des compositions dépigmentantes.

En outre, les principaux agents dépigmentants, dont l'activité se justifie par une action inhibitrice de la synthèse de la mélanine et due à un effet antagoniste sur la tyrosinase, ne manifestent qu'une action de courte durée, de telle sorte que l'on a préconisé d'associer plusieurs de ces principes actifs pour obtenir un effet plus durable et plus répétitible.

C'est ainsi que la demande de brevet PCT WO 97/02807 décrit une composition dermo-cosmétique, caractérisée en ce qu'elle contient, à titre de principe actif, une association à activité dépigmentante :
-a) d'un mélange acide comprenant :
   (i) au moins un acide α-hydroxylé ou un de ses dérivés,
   (ii) au moins un composé choisi dans le groupe comprenant l'acide kojique, l'acide caféique, l'acide azélaïque, l'acide aminobutyrique, l'acide fusarique, la 5-hydroxy 2-hydroxyméthyl-γ-pyridone, et leurs dérivés ;
- b) d'au moins un composant actif d'un extrait végétal d'au moins une plante choisie parmi : mûrier blanc, réglisse, scutelaria, pamplemousse, bouleau, bruyère, arbousier, busserole, citron, laitue, laminaire, concombre, ginseng, houblon, mais, matricaire, sauge, soja, sureau, spiruline, tilleul, aloe ferox, yukinoshita, sanguisorba, hoelen, rose fruit, α-orizanol, bumet, gingko biloba, tanlex VB, eclipsa Alba.

De préférence, l'acide ascorbique est peu utilisé et même est exclu des acides utilisables mentionnés en (a).

Parmi les dérivés des acides (i) et (ii), mentionnés dans ce document, on cite les sels, notamment les sels alcalins ou alcalino-terreux, et, en particulier les sels d'ammonium. Parmi les dérivés, on peut également citer les esters notamment les esters avec les polyols, par exemple les sucres.

On peut notamment envisager des co-esters de deux dérivés acides sur un même polyol.

Parmi les acides α-hydroxylés utilisables, il faut citer l'acide malique, l'acide citrique, l'acide lactique et l'acide glycolique ; la concentration en ces acides est avantageusement comprise entre 0,1 et 45% par rapport au poids total de la composition.

Des acides (ii) donnant des résultats particulièrement avantageux selon cette référence sont :
- l'acide kojique,
- l'acide caféique
- ou la 5-hydroxy 2-hydroxyméthyl-γ-pyridone, cette dernière étant plus particulièrement préférée.
On a donc utilisé de préférence ces composés, ou leurs sels, seuls ou en mélange, pour constituer la partie acide α-hydroxylée des compositions dépigmentantes qui agit par décapage de la couche externe cutanée.

Selon cette référence, le composant actif est de préférence apporté par un extrait végétal, obtenu à partir de mûrier blanc, réglisse, scutelaria, pamplemousse, bouleau, bruyère, arbousier, busserole, laitue, soja, aloe ferox, yukinoshira, rose fruit, Burnet, et/ou Eclipsa alba.

Des associations particulièrement avantageuses décrites ici sont celles comprenant un mélange d'acide kojique, de Burnet et d'Eclipsa alba. Ce mélange peut être combiné avec des acides α-hydroxylés et, éventuellement, avec d'autres composants d'extraits végétaux.

Lorsqu'ils sont obtenus par extraction, les principes actifs d'extraits végétaux peuvent être obtenus par une étape d'extraction en milieu hydroalcoolique à partir des tiges, des feuilles et/ou des racines de plantes. Dans la plupart des cas, l'extrait hydroalcoolique contenant le principe actif et d'autres composants pourra être utilisé tel quel, mais il est bien entendu possible de supprimer de cet extrait les composants inutiles ou néfastes, s'il en existe, et ce par des méthodes connues, telles que précipitation sélective, extraction par solvant et/ou chromatographie.

Un nouveau mécanisme d'action des produits dépigmentants a été récemment mis en évidence. Il utilise deux facteurs-clef, intervenant en amont, sur l'activité de la tyrosinase :
- le flux calcique entrant, qui module la réponse mélanique aux UV et la réceptivité membranaire aux médiateurs catécholamines agonistes α-adrénergiques ;
- sur la stabilisation de la forme inactive de la tyrosinase qui ne devient active qu'après une phosphorylation PKC-dépendante, elle-même sous l'influence de la concentration intracellulaire de Calcium.

Un troisième mécanisme d'action, également en amont de la tyrosinase, est l'intervention d'un autre antagoniste, en l'occurrence un antagoniste de l'α-MSH. L'α-MSH active la tyrosinase et conduit à une formation accrue des différentes mélanines (par exemple mélanines, phréomélanines) qui sont ensuite stockées par l'intermédiaire des mélanosomes dans les Kératinocyes et dans les Mélanocytes.

Il est donc apparu souhaitable d'inhiber encore plus complètement l'action de la tyrosinase par un antagoniste d'α-MSH. Ce type d'antagoniste de l'α-MSH présente l'avantage de manifester une efficacité supérieure aux inhibiteurs de tyrosinase conventionnels, tels que l'hydroquinone, l'arbutine, l'arbutoside, l'ascorbylphosphate de magnésium, tout en étant dépourvu d'effet cytotoxique et manifestant une excellente tolérance aux doses utilisées.

Les antagonistes de l' α-MSH manifestent une forte affinité vis-à-vis du récepteur à l'α-MSH, bien supérieure à celle des inhibiteurs connus (phénylalanine, acide undecylénique, arbutine, acide kojique, VCPMG).

Un antagoniste d' α-MSH agit également par inhibition de l'adénylaté cyclase puis après intervention de l'AMP_{C} par une inhibition de la tyrosinase, donc par une inhibition de la mélanogénèse.

L'association de trois types différents d'inhibiteurs de la tyrosinase conduit à un effet synergique, plus intense et plus prolongé.

La présente invention concerne donc une association triple constituée de trois constituant agissant sur la mélanogénèse par trois mécanismes différents :
- un inhibiteur de tyrosinase formé d'un ester d'acide kojique,
- un régulateur de flux calcique formé d'un ester de Boldine,
- un antagoniste de l' α-MSH (mélanotropine) sous la forme d'un amide de phénylalanine.

L'acide Kojique est déjà connu comme un inhibiteur efficace de la tyrosinase, mais présente des problèmes de stabilité et change de couleur avec le temps en raison de phénomènes d'oxydation. Les esters d'acide gras d'acide Kojique et spécifiquement l'ester dipalmitique d'acide Kojique de formule : 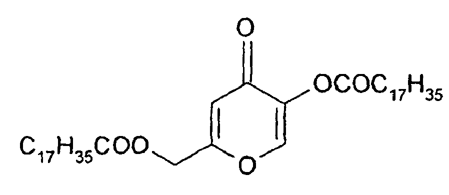 présentent une stabilité bien plus grande.

Sa dénomination chimique est 2-palmitoyloxy 5-palmitoyloxy γ-pyrone. Ce composé possède des propriétés tout à fait remarquables pour inhiber l'activité de la tyrosinase présente dans la peau humaine, de façon à inhiber la formation de mélanine (cf. brevets US 4,278,656 et 4,369,174, tous deux au nom de Sansho Pharmaceutical Co. Ltd). Il est plus efficace que l'acide Kojique. Il produit des effets remarquables dans le nuançage de la peau, en combattant les taches cutanées de mélanine dues au vieillissement, les plaques de pigmentation dues à la grossesse ainsi que, d'une manière générale, les troubles de la pigmentation cutanée de la face et du corps.

L'acide dipalmitoylkojique manifeste des propriétés dépigmentantes pour la peau plus importantes que celles de l'acide kojique. Il augmente, d'une manière plus notable que l'acide Kojique, les effets inhibiteurs sur l'activité de la tyrosinase et, de cette façon, empêche d'une manière durable la formation de mélanine.

L'acide dipalmitoylkojique possède une grande stabilité à la lumière et à la chaleur, et contrairement à l'acide kojique, il n'a pas tendance à s'oxyder avec le temps.

L'acide dipalmitoylkojique est stable sur une large plage de pH s'échelonnant de 3 à 10. On dispose ainsi d'un moyen très flexible pour la formulation des compositions.

A la différence de l'acide kojique, l'acide dipalmitoylkojique ne vire pas au brun ou au jaune au fil du temps, car il n'est pas susceptible de s'oxyder ou de se complexer avec des ions métalliques, ce qui conduit à la stabilité de la couleur. En outre, l'acide dipalmitoylkojique n'a pas d'effet chelatant alors que l'acide kojique agit principalement par chelation du cuivre nécessaire.

L'acide dipalmitoylkojique est une poudre légèrement jaune à blanc brillant, fondant entre 92 et 95 °C et présentant une teneur en principe actif de 95 % minimum. Appliqué sur la peau, il est dépourvu de propriétés irritantes.

L'acide dipalmitoylkojique est soluble dans l'huile et peut être aisément fondu à 75-85 ° C dans une phase huileuse et permet ainsi une mise en émulsion facile.

L'acide dipalmitoylkojique ne souffre pas d'incompatibilités chimiques et est compatible avec tous les écrans solaires et les conservateurs.

Un autre constituant est formé d'un agent qui agit sur la mélanogénèse. Il s'agit d'une molécule naturelle, modifiée chimiquement et sélectionnée pour agir sur les nouvelles voies récemment décrites pour la régulation de la mélanogénèse, au départ d'une approche relation-structure/activité.

Dans le mécanisme normal de stimulation de la mélanogénèse par les UV, l'ion Calcium joue un rôle important. On observe en effet une augmentation de l'ion Ca⁺ liée à une augmentation intense de la concentration en diacétylglycérol (modulation positive en amont du flux de Ca²⁺ entrant), en aval, le diacétylglycérol agit directement sur les enzymes Ca²⁺ dépendants, parmi lesquels la PKC (Phosphokinase) et les phospholipases C et D (Carlsberg 1993).

Les travaux de Buffey (1993) sur une lignée B16 soulignent le rôle important du Ca⁺⁺ pour l'efficacité de la liaison de la α-MSH, (Melanin Stimulating Hormone) à leurs récepteurs et pour la réponse positive à cette stimulation par l'activation de l'adénylcyclasc et de la pré-tyrosinase présente dans les membranes des mélanosomes.

Le Ca⁺⁺ seul (par augmentation dans le milieu extérieur) ne suffit pourtant pas à augmenter la mélanogénèse ; il agit probablement aussi sur des mécanismes intermédiaires de la régulation d'enzymes ou sur des régulations de transcription.

Ces éléments mettent en lumière les liens étroits qui existent entre les variations de taux de Calcium intracellulaire et les réponses cellulaires à la stimulation des récepteurs membranaires α -adrénergiques et à l' α-MSH.

La voie α -adrénergique est assez documentée, car elle intervient en fait sur les deux types cellulaires directement interactifs dans la mélanogénèse : les kératinocytes et les mélanocytes.

L'importance de la voie adrénergique est aussi illustrée par les travaux de Fuller (2000), qui mettent en évidence une diminution de 90 % de l'activité tyrosinase sur mélanocytes humains en présence d'antagoniste α-adrénergique. Ceci est vrai pour les mélanocytes isolés de sujets caucasiens ou à peau noire.

Au total, les publications les plus récentes montrent que la mélanogénèse « in vivo » dans la peau humaine est sous le contrôle de plusieurs facteurs, parmi lesquels le Calcium, les catécholamines issues des kératinocytes et que les récepteurs α-adrénergiques du mélanocyte semblent tous jouer des rôles pivots.

C'est à ce titre que les Demandeurs se sont intéressés à des substances d'origine naturelle et décrites pour présenter des propriétés α-adrénergiques antagonistes et perturbant aussi le flux calcique entrant : parmi celles-ci, les Demandeurs ont retenu la boldine, dont la structure est décrite comme présentant ce double effet (Eltze et al, 2002 ; Madrero et al, 1996 ; Chulia et al, 1996). On a donc synthétisé une famille de dérivés de la boldine et les Demandeurs ont exploré, parmi ces dérivés, leur capacité à réguler, d'une manière antagoniste, la mélanogénèse.

Parmi les nombreuses substances testées, la diacétyl-boldine s'est révélée être très active sur une lignée de mélanomes ainsi que sur des mélanocytes humains pour réduire réversiblement la mélanogénèse et, sur de la peau reconstruite, pour diminuer très sensiblement la pigmentation de l'épiderme.

La formule de la diacetylboldine est la suivante :

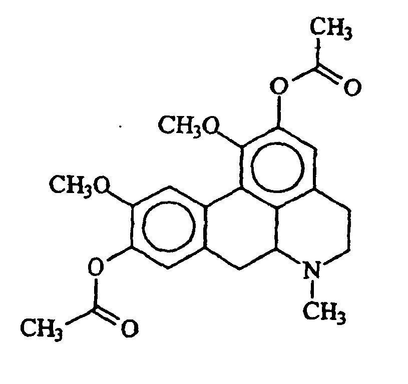

Sous sa forme commerciale, cette molécule semi-synthétique est présentée solubilisée dans un support neutre, elle est titrée à 0,1 % et est aisément incorporable dans tout type de formulation.

Les études cliniques « *in vivo* », menées sur la peau du visage de sujets asiatiques, dans les zones normales, et celle hyperpigmentée, ont mis en évidence un remarquable effet ralentisseur de pigmentation en accord avec les résultats obtenus « *in vitro.* »

Pour l'utilisation pratique de la diacetylboldine, on l'incorpore dans des triglycérides en C₈-C₁₀ dénommés, selon INCI Caprylic/caprictriglycéride (and) diacétyl-boldine.

Le troisième constituant est formé d'un antagoniste d' α-MSH qui agit très en amont de la mélanogénèse en inhibant l'adenylate-cyclase puis l'AMPc dans les mélanocytes. Il en résulte une inhibition de la Protéine kinase A (PKA) puis une inhibition de la tyrosinase.

L'inhibiteur d' α-MSH est, en l'occurrence, l'undécylènoylphénylalanine ou undécénoylphénylalanine (nomenclature INCI: undecylenoylphénylalanine) comercialisée par la firme SEPPIC sous la marque SEPIWHITE MSH®.

L'undécenoylphénylalanine est un composé pur et original. Elle se présente sous forme lipo-aminée. Elle présente une affinité cutanée élevée. Elle présente une efficacité supérieure aux molécules inhibitrices de tyrosinase de référence, testées aux dosages non cytotoxiques (hydroquinone, arbutine, acide kojique, ascorbylphosphate de magnésium). Elle manifeste une excellente tolérance aux doses d'utilisation. Elle est :
- facile à incorporer dans les formulations dépigmentantes ;
- incorporable dans tout type de formule,
- d'une parfaite stabilité,
- de présentation incolore et faiblement parfumée.

L'undécylènoylphénylalanine manifeste une affinité vis-à-vis du récepteur de α-MSH de l'ordre de 96 %, alors que la phénylalanine seule fournit une valeur de 4 %, l'acide undécylénique 5 %, l'arbutine 4 %, l'acide kojique - 24 % et le VCP MG 16 %.

On peut attribuer cette supériorité à la forme lipoaminée amphiphile.

Les compositions éclaircissantes selon l'invention contiennent ces trois constituants en quantité à peu près égale, et une proportion préférée sera de 1:1:1. Cependant, il est possible de favoriser un des modes d'action (activation des canaux calciques ou inhibition de la tyrosinase ou antagonisme vis-à-vis de la tyrosinase) en modulant les pourcentages de chacun des constituants. En pratique, on mettra davantage d'acide dipalmitoylkojique de manière à obtenir un effet dépigmentant rapide. Une telle composition contiendra de 40 à 60 % d'acide dipalmitoylkojique, de 20 à 30 % de diacetylboldine et de 10 à 40% d'undécenènoylphénylalanine.

Au contraire, s'il paraît préférable de privilégier l'antagonisme vis-à-vis de l' α-MSH, on préparera des compositions qui contiendront de 35 à 55 % d'undécenoylphénylalanine, de 20 à 40 % de diacétyl-boldine et de 10 à 45 % d'acide dipalmitoylkojique.

Les compositions selon l'invention seront présentées sous l'une des formes convenant pour l'application sur la peau, comme par exemple des crèmes, des gels, des pommades, des poudres, des lotions, des savons, des embrocations, des émulsions huile dans l'eau ou eau dans l'huile, des micro-émulsions triphasiques, des liposomes, des micro-capsules, des capsulés de crèmes, des solutions dans les solvants polaires, en sprays

Parmi ces préparations, on préfèrera les crèmes ou les gels fluides, dont l'application se fait sans problème sur toutes les parties du corps. De même, les sprays sous pression ou à la pression atmosphérique constituent des modes d'application rapides et commodes.

Les constituants sont essentiellement solubles à partir de pH6 et on pourra ainsi réaliser des compositions dont le pH est proche de celui de la peau.

Les compositions selon l'invention peuvent être additionnées d'adjuvants ou d'additifs, comme il est usuel dans les préparations cosmétiques. De tels adjuvants ou additifs sont en général des agents liants, comme l'éthylcellulose, des agents émulsionnants comme les sucrose-esters, des agents stabilisants comme la povidone, des agents anti-vieillissement, des agents conservateurs, comme les parabens, des agents antiflocculation ou antisédimentation, comme les argiles telles que la bentonite ou les zéolithes, des agents de clarification ou de turbidité comme la polyvinylpyrrolidone pontée, des agents d'aromatisation comme l'acide phénylacétique ou des agents conférant un aspect nacré comme le di-stéarate d'éthylène glycol.

Les compositions selon l'invention peuvent être en outre pigmentées avec par exemple du dioxyde de titane ou colorées avec, de préférence, des pigments végétaux, comme l'indigotine, le safran, le carthame, le carotène, l'apocaroténoate d'éthyle, la cantaxanthine ou le chlorophillinate de cuivre. On peut donc ainsi conférer aux compositions toutes les colorations ou toutes les nuances de colorations que l'on peut désirer.

Les compositions selon l'invention peuvent également être parfumées. On utilisera à cette fin des essences de lavande, l'essence de lavandin, les ionones, l'irone, le benjoin, la résine gaïac, la damascémone,ou l'essence d'Ylang-Ylang.

Des parfums semi-synthétiques ou synthétiques peuvent également convenir comme des essences florales, des concentrés d'eau de Cologne, le patchouli, les muscs artificiels, la Jasmone synthétique, la damascenoe ou l'ambrolide.

Les compositions selon l'invention sont destinées à être appliquées sur la peau, en particulier sur le visage, sur les mains, sur les bras, ou sur le buste, à raison de 1 à 4 applications par jour. La durée de ces applications est très variable, allant de 1 à 6 mois. Elle dépend principalement du type de pigmentation et de la rapidité de la dépigmentation du sujet traité.

L'invention va être plus précisément décrite dans les exemples suivants. Ils ne la limitent en aucune façon.

### Exemple 1 - Gel fluide

| | |
|---|---|
| Acide dipalmitoyl kojique | 10,0 |
| Diméthicone | 1,0 |
| Tribéhénate de glycéryle | 0,32 |
| Cyclométhicone | 3,50 |
| Trihydroxy stéarine (Thixogel RCM) | 16,0 |
| Isononanoate d'isononyle | 25,0 |
| Undécénoyl phénylalanine | 5,0 |
| Diacétylboldine à 0,1 % sur support neutre | 0,05 |
| Propylparaben | 0,20 |
| Methylparaben | 0,20 |
| Essence de lavandin | qs |
| Sphingolipides hydrophobes | 0,50 |
| Eau qsp | 100,00 ml |

On dissout les composants lipophiles (acide dipalmitoyloxy kojique, undécénoyle phénylalanine) dans l'isononanoate d'isononyle. On y ajoute les silicones et la trihydroxy stéarine. On dissout dans suffisamment d'eau les sphingolipides, les parabens, le tribéhénate de glycérine et la diacétylboldine. On incorpore progressivement la phase lipidique dans la phase aqueuse, sous agitation modérée.

Une fois le gel formé, on ajoute, toujours sous agitation, l'essence de lavandin dispersée dans une quantité équivalente de sphingolipides hydrophobes.

On obtient ainsi un gel fluide, agréablement parfumé, incolore.

### Exemple 2 - Poudre

| | |
|---|---|
| Laurate de Zinc | 1,00 |
| Dioxyde de Titane micronisé | 11,00 |
| Acide dipalmitoyl kojique | 5,00 |
| Undécenoyl phénylalanine | 5,00 |
| Silice Colloïdale | 1,40 |
| Diacétylboldine sur support | 5,00 |
| Carbonate de Calcium | 25,00 |
| Carbonate de Magnésium | 15,00 |
| Stéarate de Magnésium | 20,00 |
| Trihydroxy stéarine | 18,00 |
| Essence de Géranium Rosat | Qs |

### Exemple 3 - Crème à l'acide dipalmitoyl kojique

| | |
|---|---|
| Laurate de Zinc | 1,00 |
| Dioxyde de Titane micronisé | 1,00 |
| Acide dipalmitoyl kojique | 11,00 |
| Undécenoyl phénylalanine | 17,00 |
| Diacétylboldine sur support | 9,50 |
| Dimethicone | 5,00 |
| Tribéhénate de glycéryle | 0,68 |
| Cyclométhicone | 17,50 |
| Poudre de nylon-12 | 4,00 |
| Huile de paraffine | 22,32 |
| Stéarate de polyéthylèneglycol | 0,50 |
| Cire micro-cristalline | 0,38 |
| Eau qsp | 100 g |

### Exemple 4 - Lotion fluide pour la peau

| | |
|---|---|
| Lanoline acétylée | 2,00 % |
| Alcool stéarique | 2,50 % |
| Diméthicone | 0,25 % |
| Gomme Xanthane | 0,75 % |
| Acide dipalmitoyl kojique | 7,00 % |
| Undecenoyl phénylalanine | 7,00 % |
| Diacetylboldine sur support | 2,00 % |
| Cyclométhicone | 0,75 % |
| Bisabolol | 0,06 % |
| Stéarate de polyéthylèneglycol | 1,00 % |
| Eau | 66,00 % |
| Huile de Jojoba | 10,00 % |
| Palmitate de rétinyle | 0,50 % |
| Parfum | 0,25 % |

### Exemple 5 - Composition sèche

| | |
|---|---|
| Diméthicone | 5,0 % |
| Cétyldiméticone | 10,0 % |
| Tribehénéate de glycéryle | 0,38 % |
| Cire micro cristalline | 0,38 % |
| Acide dipalmitoyl kojique | 6,0 % |
| Undécenoyl phénylalanine | 6,0% |
| Diacetylboldine sur support | 9,0 % |
| Nylon 12 en poudre | 4,0 % |
| Thixogel RCM | 31,0% |
| Cyclométhicone | 6,97 % |
| Palmitate de rétinyle | 0,10 % |
| Talc | 30,0 % |
| Parfum | qs |

### Exemple 6 - Crème épaisse

| | |
|---|---|
| Acide dipalmitoyl kojique | 5,00 |
| Undecénoyl phénylalanine | 8,00 |
| Diacétylboldine sur support | 8,00 |
| Propylène glycol | 16,00 |
| Cyclométhicone | 6,00 |
| Glycérol | 3,00 |
| Stéarate de PEG-20 (Myrj 49) | 1,65 |
| Alcool cétylique | 1,05 |
| Triethanolamine | 1,00 |
| Carbomère (Carbopol 941®) Goodrich | 1,00 |
| Agents conservateurs | 0,50 |
| Extrait de lavande | 0,15 |
| Eau déminéralisée qsp | 100 g |

On obtient ainsi une crème consistante, que l'on peut cependant étaler à la main.

### Exemple 7 - Détermination de l'effet dépigmentant des compositions cosmétiques selon l'invention.

### Etude de la quantité de mélanine dans des épidermes reconstitués pigmentés.

Le Demandeur dispose de nombreuses formulations cosmétiques destinées à éclaircir la pigmentation cutanée.

Il a réalisé cette étude afin d'évaluer l'effet de ces produits sur la quantité de mélanine. Compte tenu de la nature des formulations à tester, l'étude a été réalisée sur des modèles d'épiderme reconstruits, pigmentés, qui ont été traités par application topique.

### MATERIELS ET METHODES

### 1. Modèle biologique

### Cytotoxicité préalable

- Epidermes : 8 épidermes mélanisés SkinEthic® (0.63 cm², 10 jours),
- Culture : milieu de culture SkinEthic, lot n° 0306011M 190 37°C, 5 %CO₂.

### Contenu en mélanine

- Epidermes : 18 épidermes mélanisés SkinEthic® (0,63 cm², 10 jours),
- Culture : milieu de culture SkinEthic®, 37°C, 5 % CO₂.

### 2. Produits à l'essai, référence

### Pour la cytotoxicité préalable:

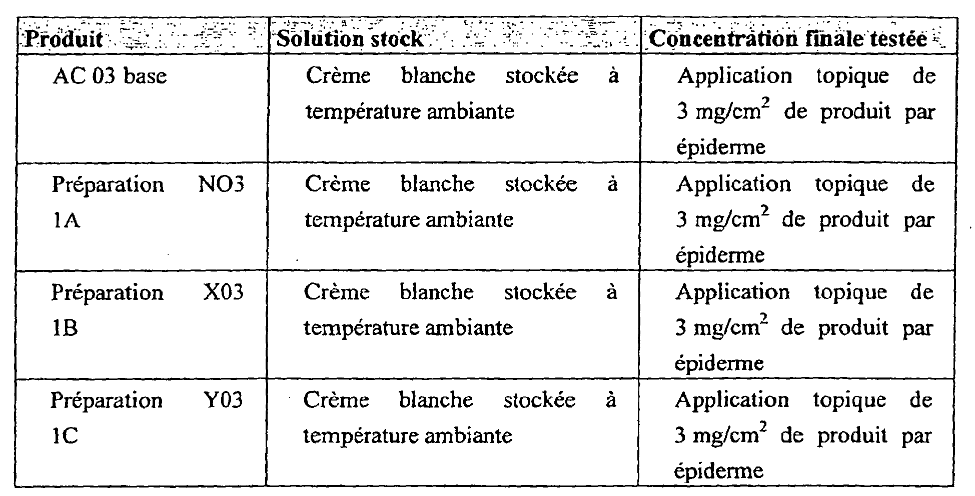

### Pour le dosage du contenu en mélanine :

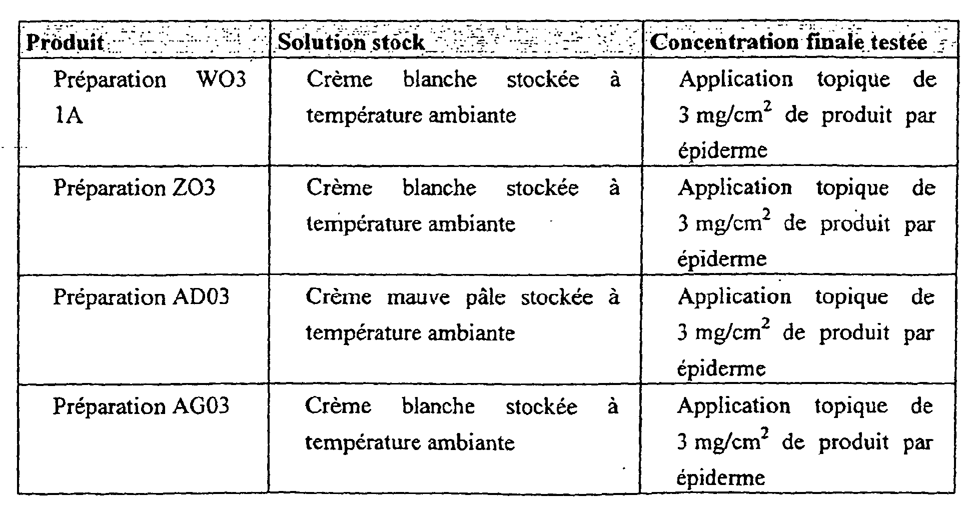

### Comparaison avec un produit de référence :

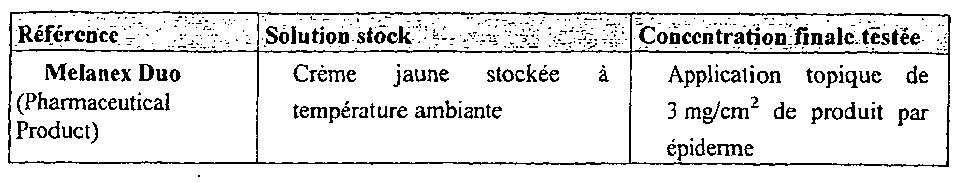

### 3. Cytotoxicité préalable sur ER

| | |
|---|---|
| Modèle biologique | 8 épidermes mélanisés SkinEthic® |
| Plaques | 12 puits |
| Pré-culture | 24 h |
| Epiderme/puits | 1 |
| Gamme de produit | Application topique de 3 mg/cm² de chaque produit pendant les 4 premiers jours |
| Réplicates | 2 |
| Contact épidermes/produit | 144 h |
| Paramètre d'évaluation | Hydrolyse en présence d'un sel de Tetrazolium (MTT) |

### 4. Dosage de mélanine

### a) traitement des épidermes

Dès réception, les épidermes ont été placés en plaques 6 puits contenant 2 ml/puits de milieu de croissance SkinEthic®, puis les produits à l'essai et la référence ont été appliqués de façon topique sur chaque épiderme (3 mg/cm² par épiderme, 3 épidermes par traitement), le contact avec les produits a été réalisé pendant 6 jours avec renouvellement du traitement des épidermes chaque jour pendant les 4 premiers jours. Trois épidermes « témoin » non traités ont été réalisés en parallèle. Après traitement, les épidermes ont été rincés avec du tampon PBS découpés et le dosage de mélanine à été réalisé.

### b) Dosage de mélanine

Le dosage de mélanine a été réalisé suivant le protocole décrit par SkinEthic. Les épidermes ont été rincés en PBS, découpés et transférés en tubes Eppendorf. La mélanine a été extraite avec une solution eau/toluène à 90° C pendant 45 minutes. Les extraits ont été transférés en plaque 96 puits (2 puits pour chaque extrait) et la densité optique (DO) à 405 nm a été mesurée sur un lecteur de plaques (SoftMax, Molecular Devices). La quantité de mélanine (en µg/ml) a été déterminée en utilisant une gamme de concentrations de mélanine (courbe standard).

### 5. Traitement des données

- Les données ont été transférées et traitées sous logiciel PRISM® (Graph Pad Software).
- Les comparaisons intergroupes ont été réalisées par analyse de variance (ANOVA) à l'aide du test de comparaison multiple de Dunnett.

### RESULTATS ET CONCLUSIONS

### 1. Cytotoxicité préalable :

### Tableau 1 :

Les résultats de viabilité obtenus ont permis au Demandeur de choisir les produits à tester pour la suite des essais.

### 2. Dosage de la mélanine

La crème de référence « Melanex duo » a diminué la quantité de mélanine produite par les épidermes reconstruits mélénisés (83 % du témoin, diminution de pigmentation de 17 %).

Ce résultat attendu valide l'essai

Les produits d'essai W031A" et ZO3" ont légèrement diminué la quantité de mélanine produite par les épidermes reconstruits mélanisés. Cependant, cet effet n'était pas statistiquement significatif.

Les produits d'essai AD03" et AG03" ont diminué la quantité de mélanine produite par les épidermes reconstruits mélaninés (respectivement 84 % et 82 % du témoin non traité, p<0,05).

L'analyse statistique n'a pas montré de différence significative entre les effets des produits à l'essai et ceux du produit de référence.

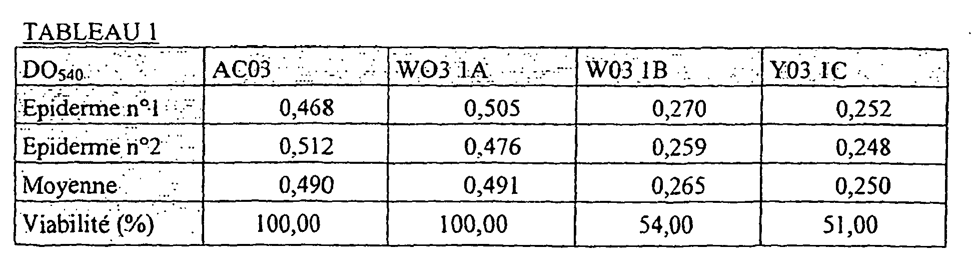

Tableau 1 : Cytotoxicité préalable réalisée sur épidermes reconstruits mélanisés mis en contact avec les produits pendant 144 heures (avec 4 applications successives du produit). Viabilité exprimée en pourcentage par rapport à la base. Révélation par le test au sel de Tétrazolium (MTT).

### Quantité de mélanine produite par les épidermes

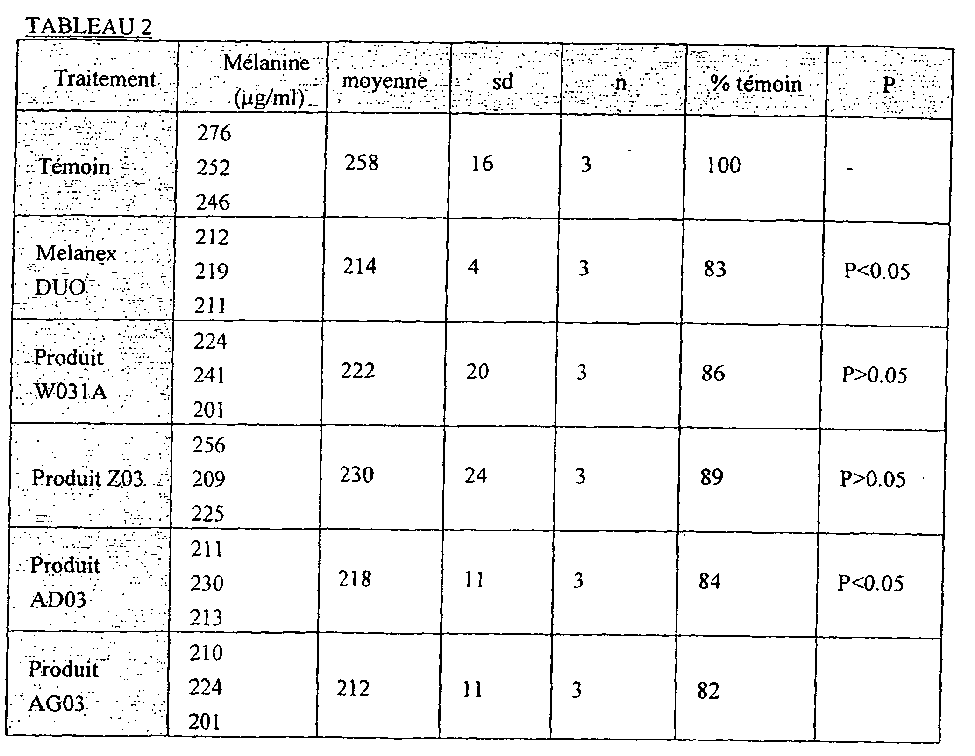

### Quantité de mélanine

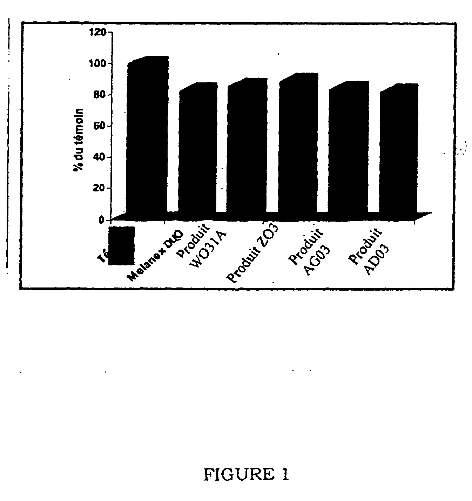

La figure 1 jointe en annexe fait apparaître les effets des différents traitements sur la quantité de mélanine produite par les épidermes mélanisés.

## Revendications

1. Compositions dépigmentantes à actions multiples et prolongées, **caractérisée en ce qu'**elles renferment une association triple de trois constituants agissant sur la mélanogénèse :
a) un ester d'acide kojique,
b) un ester de boldine
c) une amide de phénylalanine,
associée à un excipient ou à un véhicule inerte, approprié pour l'application topique.

2. Compositions dépigmentantes selon la revendication 1,
dans lesquelles l'ester d'acide kojique est un ester d'acide gras d'acide kojique.

3. Compositions dépigmentantes selon la revendication 1 ou la revendication 2,
dans lesquelles l'ester d'acide kojique est un ester dipalmitique.

4. Compositions dépigmentantes selon la revendication 1,
dans lesquelles l'ester de boldine est la diacétylboldine.

5. Compositions dépigmentantes selon la revendication 1,
dans lesquelles l'amide de phénylalanine est l'undécénoylphénylalanine.

6. Compositions dépigmentantes selon la revendication 1,
dans lesquelles les trois constituants sont présents en quantité à peu près égale.

7. Compositions dépigmentantes selon la revendication 1,
dans lesquelles de 40 à 60 % d'acide dipalmitoyloxy kojique sont présents.

8. Compositions dépigmentantes selon la revendication 1,
dans lesquelles de 20 à 30 % de diacétylboldine sont présents.

9. Compositions dépigmentantes selon la revendication 1,
dans lesquelles de 10 à 40 % d'undécénoylphénylalanine sont présents.

10. Compositions dépigmentantes selon la revendication 1,
qui contiennent
de 35 à 45 % d'undécenoyloxy phénylalanine, de 20 à 40 % de diacétylboldine et de 10 à 45 % d'acide dipalmitoyl kojique rapporté au mélange, l'ensemble formant 100% des principes actifs.

11. Compositions dépigmentantes selon l'une des revendications précédentes, **caractérisés en ce qu'**
elles sont présentées sous l'une des formes convenant pour l'application sur la peau.

12. Compositions dépigmentantes selon la revendication 11,
**caractérisées en ce qu'**
elles sont présentées sous forme de crèmes, de gels fluides ou épais ou de sprays.

13. Compositions dépigmentantes selon l'une des revendications précédentes,
dans lesquelles on incorpore un ou plusieurs adjuvants ou additifs.

14. Utilisation des compositions dépigmentantes selon l'une des revendications 1 à 13,
en vue de l'application sur la peau d'une telle composition à raison de 1 à 4 applications par jour en vue d'obtenir une dépigmentation durable de l'épiderme.

## Claims

1. Depigmenting compositions with multiple and protraded activity, **characterized in that** they contain an association of three components acting on melanogenesis :
a) a kojic acid ester,
b) a boldine ester
c) a phenylalanin amide

2. Depigmenting compositions according to claim 1, in which the kojic acid ester is a fatty acid ester of kojic acid.

3. Depigmenting compositions according to claim 1 or claim 2, in which the kojic acid ester is a dipalmitic ester.

4. Depigmenting compositions according to claim 1, in which the boldine ester is diacetylboldine.

5. Depigmenting compositions according to claim 1, in which the phenylalanin amide is undecenoylphenylalanin.

6. Depigmenting compositions according to claim 1, in which the three components are present in about the same amounts.

7. Depigmenting compositions according to claim 1, containing from 40% to 60% of dipalmitoyloxykojic acid.

8. Depigmenting compositions according to claim 1, containing from 20% to 30% of diacetylboldine.

9. Depigmenting compositions according to claim 1, containing from 10% to 40% of undecenoylphenylalanin.

10. Depigmenting compositions according to claim 1, containing from 35% to 45% undecenoylphenylalanin, from 20% to 40% diacetylboldine and from 10% to 45% dipalmitoyloxykojic acid in relation to the mixture, which forms 100% of the active principles.

11. Depigmenting compositions according to any of the previous claims, **characterized in that** they are in a form suitable for application on the skin.

12. Depigmenting compositions according to claim 11, **characterized in that** they are in the form of creams, fluid or thick gels and sprays.

13. Depigmenting compositions according to any of the previous claims, in which one or more adjuvants or additives are presents.

14. Use of depigmenting compositions according to any of claims 1 to 13, for topical application 1 to 4 times a day, to obtain a long epidermis depigmentation.

## Patentansprüche

1. Depigmentierende Zubereitungen mit vielfachen und verlängerten Wirkungen, **dadurch gekennzeichnet, daß** sie eine Zusammensetzung von drei Komponenten, die auf Melanogenesis handeln, enthält :
a) ein Kojylsaüreester
b) ein Boldineester
c) ein Phenylalaninamide
verbindet mit einem bewegungslos, geeignet für Hautauftragung, Bindemittel.

2. Depigmentierende Zubereitungen nach Anspruch 1, in denen der Kojylsaüreester ein Kojylfettsaüreester ist.

3. Depigmentierende Zubereitungen nach einem der Ansprüche 1 oder 2, in denen der Kojylsaüreester ein Dipalmitylsaüreester ist.

4. Depigmentierende Zubereitungen nach Anspruch 1, worin der Boldineester Diacetylboldine ist.

5. Depigmentierende Zubereitungen nach Anspruch 1, worin der Phenylalaninamide Undecenoylphenylalanine ist.

6. Depigmentierende Zubereitungen nach Anspruch 1, worin die Quantitäten der drei Komponenten fast gleich sind.

7. Depigmentierende Zubereitungen nach Anspruch 1, enthaltend von 40 bis 60 Gewichts-% Dipalmitoyloxykojylsaüre.

8. Depigmentierende Zubereitungen nach Anspruch 1, enthaltend von 20 bis 30 Gewichts-% Diacetylboldine.

9. Depigmentierende Zubereitungen nach Anspruch 1, enthaltend von 10 bis 40 Gewichts-% Undecenoylphenylalanine.

10. Depigmentierende Zubereitungen nach Anspruch 1, enthaltend von 35 bis 45 Gewichts-% Undecenoylphenylalanine, von 20 bis 40 Gewichts-% Diacetylboldine und 10 bis 45 Gewichts-% Dipalmitoyloxykojylsaüre, die Verbindung 100 Gewichts-% von den Wirkstoffen ausmachend.

11. Depigmentierende Zubereitungen nach einem der Ansprüche 1 bis 10, **dadurch gekennzeichnet, daß** sie in einer geeignet für die Hautauftragung Form sind.

12. Depigmentierende Zubereitungen nach Anspruch 11, **dadurch gekennzeichnet, daß** sie in Form von Cremes, flüssigen oder dickflüssigen Gelen oder Sprays sind.

13. Depigmentierende Zubereitungen nach einem der Ansprüche 1 bis 12, worin ein oder mehrere Zusatz- oder Zuschlaftstoffe zugesetzt sind.

14. Verwendung der depigmentierende Zubereitungen nach einem der Ansprüche 1 bis 13, für der Hautauftragung einer solchen Zusammensetzung, nach einem Rat von 1 bis 4 täglichen Applikationen, mit dem Vorsehen einer langen Epidermis Depigmentierung.
